(19) Europäisches Patentamt
European Patent Office
Office européen des brevets

(11) EP 4 388 977 A1

(12) **EUROPEAN PATENT APPLICATION**

(43) Date of publication:
**26.06.2024 Bulletin 2024/26**

(21) Application number: **22214490.9**

(22) Date of filing: **19.12.2022**

(51) International Patent Classification (IPC):
***A61B 3/02*** *(2006.01)*

(52) Cooperative Patent Classification (CPC):
**A61B 3/022**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **medignition AG**
**8004 Zürich (CH)**

(72) Inventors:
• **LIENHARD, Kenny R.**
**5000 Aarau (CH)**
• **FAES, Livia**
**8032 Zürich (CH)**
• **BACHMANN, Lucas M.**
**8002 Zürich (CH)**

(74) Representative: **Bremi, Tobias Hans**
**Isler & Pedrazzini AG**
**Giesshübelstrasse 45**
**Postfach 1772**
**8027 Zürich (CH)**

(54) **METHOD FOR MEASURING THE SPATIAL CONTRAST SENSITIVITY OF A USER**

(57) Method for measuring the spatial contrast sensitivity of an eye of a user, wherein said method, by way of an electronic device (1) with a display (2), exposes the eye of the user to a series of test pictures (3), said series comprising at least one first subseries, in which a sequence of differing grating patterns (4,5) is shown sequentially, each element of the sequence either aligned in a vertical direction (6), or aligned inclined in an inclined direction (7) from the vertical direction to the left or to the right by a minimum angle ($\alpha$) of at least 5°, and wherein the user, depending on the user's visual perception, has to enter whether the perception is a vertical orientation, or an inclined direction to the left or an inclined direction to the right, to move to a next grating pattern of the sequence, and wherein along the sequence the contrast amplitude of the sinusoidal grating pattern (4,5) is successively decreasing

**FIG. 1**

EP 4 388 977 A1

**Description**

TECHNICAL FIELD

The present invention relates to a method for measuring the spatial contrast sensitivity of an eye of a user as to corresponding computer implementations and devices.

PRIOR ART

The measurement of the spatial contrast sensitivity of the human eye is an important tool to determine or monitor the performance of the eyes of a human and also in some situations to monitor or detect or supervise pathologic changes in the eye or the development of treatment of the eye.
For example Cataract, which refers to the opacification of the eye lens, has been highlighted as the major cause of preventable visual impairment by the World Health Organisation. Once diagnosed, the lens may be exchanged by an artificial intraocular lens in a surgical outpatient procedure, thereby restoring sight. Today, the identification of cataract is resource intense, affording highly specialized workforce (i.e., ophthalmologists or optometrists) and expensive technology (i.e., slit lamp, medical cameras). This may contribute to the substantial barrier in care delivery for patients with cataracts worldwide. For screening purposes, particularly in low- and middle-income countries, it would be impactful to have affordable and portable screening tools which are valid in the hands of non-medical specialist care providers, or patients.
While common in daily medical routine, the monitoring of visual function in terms of visual acuity measurement, has been shown to lack sensitivity and specificity for the identification of cataract. (Best-corrected) visual acuity, defined as angular resolution power of the eye, is affected in rather late stages of cataract. Contrast sensitivity on the other hand, is known to be affected in early stages of cataract due to an increase of light scattering and absorption caused by the lens opacification. Contrast sensitivity is in the present context understood to be a measure of the ability to discern between luminance of different levels and is a complementary visual function to acuity which is measured at maximal (black-and-white) contrast. As most everyday visual stimuli are of low contrast, contrast sensitivity reflects daily visual function of cataract patients more accurately than visual acuity measurement.

SUMMARY OF THE INVENTION

It is therefore an object of the present invention to provide an improved method for the measurement of the spatial contrast sensitivity of the human eye.
The sole measurement of a monochromatic illuminance contrast sensitivity (black-and-white) may be affected various factors so also by other factors than lens opacification such as age, retinal photoadaptation or eye diseases other than cataract (i.e., Fuchs endothelial dystrophy or age-related macular degeneration). Since light scattering and absorption by lens opacifications preferentially occur in short wavelengths (tritan spectrum), a differential decrease in the contrast sensitivity function along the tritan spectrum, provides additional specificity and for example a possible basis for a more robust signal for the identification of cataract.
Therefore what is proposed is a digital task that assesses a monochromatic illuminance (black-and-white) and preferably also a colour based, in particular tritan (blue-and-yellow) contrast sensitivity for different spatial frequencies, thereby generating individual sensitivity thresholds and functions.
According to a first aspect of the present invention, it relates to a method for measuring the spatial contrast sensitivity of an eye of a user.
Said method, by way of an electronic device with a display, exposes the eye of the user to a series of test pictures.
This series comprises at least one first subseries, in which a sequence of differing grating patterns is shown sequentially, each grating pattern of the sequence either aligned in a vertical orientation, or
aligned inclined in an inclined orientation (7) from the vertical direction

to either the left or
to the right

by a minimum angle ($\alpha$) of at least 5°, and wherein along the sequence the orientation of the grating patterns (4, 5) is, preferably randomly, varied.
The user, depending on the user's visual perception, has to enter whether the perception is a vertical orientation, or an inclined direction to the left or an inclined direction to the right, to move to a next grating pattern of the sequence.
Along the subseries the contrast amplitude of the grating pattern is successively decreasing. According to a first preferred embodiment, the series comprises at least one further second subseries, in which a sequence of grating patterns is shown, the modulation of which is different from the one of the first subseries, wherein preferably this difference is a difference in spatial grating frequency and/or in colour. Preferably, a first subseries is a monochromatic series, with black/white or rather grey/grey grating, and the second subseries is a non-monochromatic, i.e. bi-colour grating sequence.

Correspondingly, according to a further preferred embodiment the grating patterns of the first subseries monochromatically modulate in each grating pattern between black and white and/or a first grey tone and a second grey tone lighter than the first grey tone, and along the first subseries with successively decreasing contrast, and wherein the grating patterns of the second subseries modulate non-monochromatically in each grating pattern between a first colour and a second colour different from the first colour, and along the second subseries with successively decreasing contrast, and wherein either the first and second subseries are carried out sequentially or intermittently.

Preferably the subseries are carried out sequentially, so for example the first subseries is carried out first until reaching a final display without any contrast, i.e. both lines having the same shading as typically the background of the display, and then the second subseries is carried out again until reaching a final display without any contrast, i.e. both lines having the same shading as the background of the display.

Preferably, for the second subseries said first colour is blue and said second colour is yellow.

According to yet another preferred embodiment, the first subseries and the second subseries each comprise in the range of 5-50, preferably in the range of 20 - 30 grating patterns which are decreasing in contrast, wherein preferably the last grating pattern of each subseries has no contrast.

As a measure of the spatial contrast sensitivity of the user, the difference between successful identification of correct grating pattern orientation between the first and the second subseries can be preferably taken.

A score of 1 can be assigned if the user identifies the direction of a grating pattern correctly, and a score of zero can be assigned if the user identifies the direction of a grating pattern incorrectly, and wherein the scores are numerically evaluated, preferably summed up along a series and/or subseries.

A difference in summed up scores of the first subseries relative to the summed up scores of the second subseries of more than 1, preferably of more than 2 or more than 3, is preferably taken as a significant indication of reduced spatial contrast sensitivity of the user. Further preferably that difference is in the sense that the summed up scores of the first monochromatic subseries is larger than the summed up scores of the second non-monochromatic subseries.

Further it is possible to take account of the time taken by the user to actually identify a direction of the grating pattern for the control of the series but also for the evaluation of the performance of the user.

The subseries is normally automatically terminated if the user has failed incorrectly identifying the orientation for at least one grating pattern. It is also possible to only terminate after two successive inputs have been wrong, or it is possible to go back in contrast to finally sample around the actual threshold value of the user.

Preferably, the electronic device is a portable electronic device, and preferably the display is a touch sensitive display. Preferably the electronic device is a mobile phone or tablet. Typically and advantageously the grating patterns are sinusoidal grating patterns.

The electronic device further preferably comprises at least one camera facing in the direction of the display and of the user. Such a camera can be used for controlling the method.

Such a camera driven control may comprise at least one or a combination of the following:

- control of the distance of the eye of the user to the display,
- control of the open eye of the user,
- control of the identity of the user, preferably by using face recognition.

Preferably the output of the camera is used for controlling at least one of the following parameters of the method: subseries, type and number per subseries of grating patterns (colour, monochromatic), spatial frequency of the grating patterns, temporal speed of switching between grating patterns along the subseries, degree of change of contrast between grating patterns between grating patterns.

Typically, the contrast amplitude of the sinusoidal grating pattern within each subseries is successively decreasing, and wherein this decrease is linear along each full subseries. Alternatively it can be linear in an initial phase until reaching a switch point, preferably statically determined or as a function of the behaviour of the user, and subsequently changed to a different less steep linear or non-linear decrease.

The spatial frequency of the preferably sinusoidal grating patterns normally is in the range of 0.01-5 lines/mm, preferably in the range of 0.3-3 lines/mm, these values preferably giving for a viewing distance of about 30 cm and wherein preferably within one subseries or within all subseries the spatial frequency is constant.

On the display where the grating patterns are displayed, there are preferably displayed input fields or buttons for the user to touch manually for signing the respective grating pattern a perceived orientation.

Preferably there is at least one button for indicating a vertical orientation, preferably located above the grating pattern, a separate button for indicating a left orientation, preferably located to the left of the grating pattern, and a separate button for indicating a right orientation, preferably located to the right of the grating pattern, or a single slide button for inputting the perceived orientation can also provided.

The grating pattern is preferably rectangular, square, oval or circular field, most preferably it is a circular field of constant spatial frequency.

Furthermore the present invention relates to a data processing program for carrying out a method as described above on a preferably portable electronic device having a display and a data evaluation unit, wherein the display is preferably a touch-sensitive display, and wherein furthermore preferably the electronic device additionally has a camera directed towards the user, wherein the display grating patterns is two- or three-dimensional, preferably in the form of a mobile application.

Further embodiments of the invention are laid down in the dependent claims.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

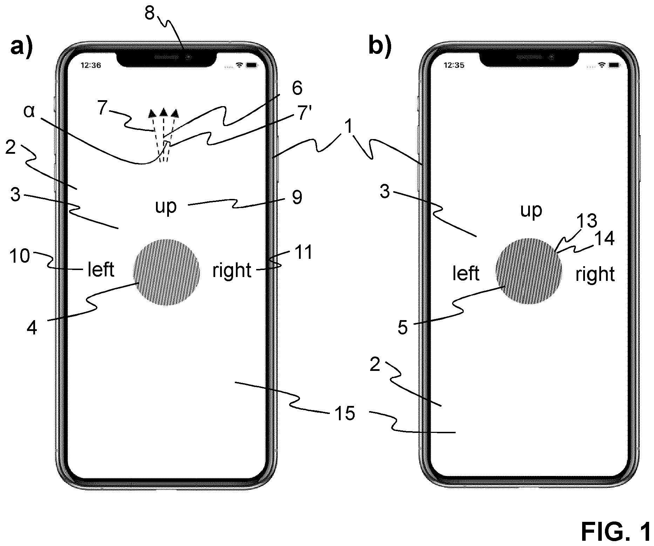

Fig. 1    shows a mobile phone with grating patterns and corresponding input fields, wherein in a) a monochromatic grating pattern is shown tilted to the right, and in b) a blue/yellow modulating grating pattern is shown tilted to the right.

DESCRIPTION OF PREFERRED EMBODIMENTS

**Task**

The task is a digital version of a test for spatial contrast sensitivity assessments in near distance (30cm testing distance). Contrast sensitivity is measured using sinusoidal grating patterns which vary in frequency and contrast. The number of light-dark cycles of the grating that subtend 1-degree visual angle is a measure of the spatial frequency of the grating, expressed in cycles per degree (cpd). The human visual system is able to detect spatial frequencies up to about 60 cpd. There is no lower limit, but generally measurements are not made below about 0.1 cpd. A typical test tests spatial frequencies of 1.5, 3, 6, 12 and 18 cpd. The contrast of a sinusoidal grating is based on maximum and minimum luminance. It is a dimensionless variable having values ranging from 0 (uniform field) to 1.0. The minimum contrast at which a grating can be distinguished from a uniform field with some fixed level of accuracy is the contrast threshold. The reciprocal of threshold contrast is called contrast sensitivity. The contrast sensitivity function is obtained by measuring contrast thresholds over a range of spatial frequencies.

In the exemplified task, there are two different task types as subseries: monochromatic (black-and-white) and tritan (blue-and-yellow). For each task type/subseries, 15 or 25 circles are drawn. We show a user one circle at a time (see **Fig. 1**).

In **Fig. 1** a mobile phone is illustrated as electronic device 1, having a display 2, which in this case is a touch sensitive display. On the display in the illustration in a) a monochromatic grating pattern 4 is illustrated having a circular shape. The lines are actually a sinusoidal grating pattern with constant frequency over the full surface of the circular shaped grating pattern 4. The vertical direction is defined as the normal holding direction of the device and is illustrated by way of arrow 6. The grating pattern 4 is not oriented in that vertical direction but is inclined by an angle $\alpha$ in this case to the right along an right inclination direction 7'. Around that grating pattern 4 there are input buttons, specifically above the grating pattern 4 there is a button designated as "up", and if the user touches that button it means the user wants to give feedback that the pattern is oriented vertically, i.e. the lines are oriented parallel to arrow 6.

There is, to the left of the grating pattern 4, a corresponding button 10 designated as "left", for the user to input if the perception is that the grating pattern is oriented to the left along schematically illustrated arrow 7.

Further to the right of the grating pattern 4, there is a corresponding button 11 designated as "right", for the user to input if the perception is that the grating pattern is oriented to the right along schematically illustrated arrow 7'. This would be the correct input for the illustrated situation in **Fig. 1a.**

It is noted that when in the general context of this document mention is made of a black and white grating pattern which is decreased in contrast, this means that the corresponding lines only oscillate strictly speaking between black and white for the maximum contrast illustration, while for any illustration with less contrast the black lines successively become a lighter grey tone, and the white lines successively become a darker grey tone until, in the final display, the grey tone of the "black" and of the "white" lines are identical.

The corresponding situation for the second subseries which is in colour as illustrated in **Fig. 1b,** in this case there are no black and white or, when decreasing in contrast, dark and light grey lines, but there are blue lines 13 and yellow lines 14, the colour contrast of which along the second subseries is successively reduced to reach the value of the background display colour/grey shading 15.

Furthermore the device is equipped with a camera 8, that camera can be used to control the process, for example to identify the user by face recognition, to identify the distance of the user and/or the eye which is actually open during the process and also to give visual or acoustic or vibration feedback to the user if along a series the wrong eye is open or both eyes are open, or to indicate that the distance of the eye to the device should be adapted. Alternatively the camera can be used to adapt the line frequency to the distance of the eye to the device. Also the camera can be used to identify the general brightness in the surrounding to correspondingly adapt the brightness of the display or the colour shading of the display.

The device typically has means to communicate with a central database, for example by a mobile telecommunications network, to record the actual performance of the user, for example if the performance of a user is to be monitored and statistically evaluated or to be forwarded to corresponding authorities if certain changes in the performance are detected. To draw the circle, in this case we use 1/3 of the screen width. The circle is filled with a sinusoidal grating pattern. Line width is either fixed or is specifically calculated in 3 steps:

Step 1: line width in millimeter where y equals distance between eye and mobile device

$$x = \tan(0.5) * y / 12$$

Step 2: convert millimeters to pixels where z equals device resolution in points per pixel (PPI)

$$a = (z * x) / 25.4$$

Step 3: adapt line width to user's best corrected visual acuity (v)

$$p = a + ((1 - v) * a)$$

The lines are colored black-and-white (monochromatic) and blue-and-yellow (tritan) respectively and vary in contrast. Two colors are defined for each circle. A color is either black-and-white (monochromatic task) or blue-and-yellow (tritan task).

For the blue and yellow subseries in case of 15 patterns to be shown to the user, relative to a background the following colour sequence can be used:

| c1 (yellow) | c2 (blue) | background |
| --- | --- | --- |
| rgb(195, 169, 14) | rgb(61, 176, 255) | rgb(171, 171, 171) |
| rgb(195, 169, 37) | rgb(81, 175, 255) | rgb(171, 171, 171) |
| rgb(194, 169, 52) | rgb(95, 175, 255) | rgb(171, 171, 171) |
| rgb(193, 169, 65) | rgb(107, 174, 255) | rgb(171, 171, 171) |
| rgb(192, 170, 76) | rgb(117, 174, 255) | rgb(171, 171, 171) |
| rgb(190, 170, 87) | rgb(126, 174, 253) | rgb(171, 171, 171) |
| rgb(189, 170, 97) | rgb(133, 173, 244) | rgb(171, 171, 171) |
| rgb(187, 170, 107) | rgb(140, 173, 235) | rgb(171, 171, 171) |
| rgb(186, 170, 116) | rgb(146, 173, 225) | rgb(171, 171, 171) |
| rgb(184, 170, 125) | rgb(151, 172, 216) | rgb(171, 171, 171) |
| rgb(182, 170, 135) | rgb(156, 172, 207) | rgb(171, 171, 171) |
| rgb(179, 170, 144) | rgb(160, 172, 198) | rgb(171, 171, 171) |
| rgb(177, 171, 153) | rgb(164, 172, 189) | rgb(171, 171, 171) |
| rgb(174, 171, 162) | rgb(168, 171, 180) | rgb(171, 171, 171) |
| rgb(171, 171, 171) | rgb(171, 171, 171) | rgb(171, 171, 171) |

The user must determine for each circle or grading pattern, whether the lines are rotated to the left (-10 degree), to the right (+10 degree), or up / down (0 degree).

For each circle, we change the contrast between the lines. The lower the contrast, the more difficult for the user to determine line rotation. A user can touch the circle if she is unable to see a contrast between the lines.

The background is colored in gray. Each eye is assessed monocularly at 30 cm distance in photopic conditions on a mobile device.

**Score**

To calculate the score, we assess a user's performance in recognizing line directions on the monochromatic (black-and-white) and tritan (blue-and-yellow) task fields. To assess the performance, we assign a score of 1 if a user identifies line direction correctly. Specifically, we investigate the differences between the monochromatic and the tritan score. While one subject with normal high-performance reaches nearly the maximum score for both task fields (i.e., the difference is nearly 0), a subject with reduced performance (e.g. indicative of cataract) will perform worse in the tritan task field (i.e., the difference is clearly above 0). Intensive data analysis has shown that the proposed scheme not only provides for a very accurate determination of the spatial contrast sensitivity of a user, surprisingly the automated method using an electronic device leads to a much higher reliability of the determine spatial contrast sensitivity than if the user is faced with similar patterns in a sequence of physical pictures. While not being bound to any theoretical explanation, this seems to be associated with the much higher focus that is possible using an electronic display, associated with the much less changing surrounding when carried out in a digital way, and associated with the superior quality of the graphical representations on display.

**Cookbook:**

1. A user selects the eye that she aims to test via a button labeled left or right eye. Alternatively, the eye is selected via face recognition. The user closes the eye that is not tested and leaves the tested eye open. The face recognition algorithm selects the eye to test and shows the test screen.

2. The app presents the test screen (see **Fig. 1**). The app starts with the monochromatic (black-and-white) task. A circle is drawn. The circle is filled with lines forming the grating pattern. Line color is successively modified (see columns c1 and c2 in color sheet above). We start with the color set having the highest contrast and continuously decrease the contrast with each circle.

3. Line width is calculated as follows:

a. (tan 0.5) x DISTANCE_BETWEEN_EYE_AND_DEVICE = X,
where we take a constant distance of 300 millimeters
b. X : 12 = line width in millimeters
c. Convert millimeters to pixels and round no nearest pixel

4. The lines are randomly rotated by -10deg, 0deg, or 10deg.

5. The user must indicate line direction via up, left, or right button. The user can tap the circle if there is no visible contrast.

6. The app gives the user feedback whether the selected line direction was right or wrong. If the line direction was correct, a score of 1 is attributed for the monochromatic task. Otherwise, the score for the current circle is 0.

7. The app automatically continues with the next color set. In total, 25 circles are presented during the monochromatic task.

8. The app calculates total score for the monochromatic task. Maximum score is 25.

9. The app continues with the tritan task. Again, 25 circles are presented, and the user must indicate the correct line direction. We start with the color set having the highest contrast and continuously decrease the contrast with each circle. The app calculates total score for the monochromatic task. Maximum score is 25.

**Technical requirements**

The digital task is compatible with mobile devices (i.e., smartphones and tablets). The mobile device should have a minimum screen size of 320 x 480 pixels. The circular task field is 1/3 of the screen width. The device orientation is portrait mode. A user can indicate the direction of the lines via touch interaction by using one of the four buttons (left, right, up/down) placed around the circular task field. If the user does not recognize a contrast between the lines and is therefore unable to indicate the line direction, she can press on the circle.

We control the screen brightness during task execution by setting a mobile device's brightness preferably to its maximum level. This overwrites a user's default brightness settings including auto-brightness set by the operating system. Further, we control for display sizes and resolutions by relying on the following concepts. When we use the unit pixels, we refer to Cascading Style Sheets (CSS) pixels which are interpreted as points (on iOS) and device independent pixels (on Android). The size of a CSS pixel, a point, and a device independent pixel is always the same regardless of the phone and the screen it is on. An iOS point is equivalent to 1/163 of an inch, a device independent pixel is similar to an iOS point and is equivalent to 1/160 of an inch. We use the term pixels to refer to CSS pixels which are interpreted as points (iOS) and device independent pixels (Android) respectively.

LIST OF REFERENCE SIGNS

| 1 | mobile phone, tablet device | 10 | input button left |
|---|---|---|---|
| 2 | display | 11 | input button right |
| 3 | test picture, | 13 | blue line |
| 4 | grating pattern, black and white | 14 | yellow line |
| | | 15 | background colour/grey tone of the display |
| 5 | grating pattern, colour | | |
| 6 | vertical direction | | |
| 7 | inclined direction, left | $\alpha$ | inclination angle of grating |
| 7' | inclined direction, right | | pattern relative to vertical |
| 8 | camera | | direction |
| 9 | input button up | | |

## Claims

1. Method for measuring the spatial contrast sensitivity of an eye of a user,

   wherein said method, by way of an electronic device (1) with a display (2), exposes the eye of the user to a series of test pictures (3),
   said series comprising at least one first subseries, in which a sequence of differing grating patterns (4,5) is shown sequentially, each grating pattern (4, 5) of the sequence either aligned in a vertical orientation (6), or aligned inclined in an inclined orientation (7) from the vertical direction to either the left or to the right by a minimum angle ($\alpha$) of at least 5°, and wherein along the sequence the orientation of the grating patterns (4, 5) is, preferably randomly, varied and
   wherein the user, depending on the user's visual perception, has to enter whether the perception is a vertical orientation, or an inclined direction to the left or an inclined direction to the right, to move to a next grating pattern of the sequence, and
   wherein along the subseries the contrast amplitude of the grating pattern (4,5) is successively decreasing.

2. Method according to claim 1, wherein the series comprises at least one further second subseries, in which a sequence of grating patterns (4,5) is shown, the modulation of which is different from the one of the first subseries, wherein preferably this difference is a difference in spatial grating frequency and/or in colour.

3. Method according to claim 2, wherein the grating patterns (4) of the first subseries monochromatically modulate in each grating pattern (4) between black and white and/or a first grey tone and a second grey tone lighter than the first grey tone, and along the first subseries with successively decreasing contrast, and

   wherein the grating patterns (5) of the second subseries modulate non-monochromatically in each grating pattern (5) between a first colour and a second colour different from the first colour, and along the second subseries with successively decreasing contrast, and
   wherein either the first and second subseries are carried out sequentially or intermittently.

4. Method according to claim 3, wherein said first colour is blue and said second colour is yellow.

5. Method according to any of the preceding claims 3 or 4, wherein the first subseries and the second subseries each comprise in the range of 5-50, preferably in the range of 20 - 30 grating patterns (4, 5) which are decreasing in contrast, wherein preferably the last grating pattern (4, 5) of each subseries has no contrast.

6. Method according to any of the preceding claims 3 - 5, wherein as a measure of the spatial contrast sensitivity of the user, the difference between successful identification of correct grating pattern orientation between the first and the second subseries is taken.

7. Method according to any of the preceding claims, wherein a score of 1 is assigned if the user identifies the direction of a grating pattern (4, 5) correctly, and a score of zero is assigned if the user identifies the direction of a grating pattern (4, 5) incorrectly, and wherein the scores are numerically evaluated, preferably summed up along a series and/or subseries.

8. Method according to claim 6 and claim 7, wherein a difference in summed up scores of the first subseries relative to the summed up scores of the second subseries of more than 1, preferably of more than 2 or more than 3, is taken as a significant indication of reduced spatial contrast sensitivity of the user, wherein preferably that difference is in the sense that the summed up scores of the first monochromatic subseries is larger than the summed up scores of the second non-monochromatic subseries.

9. Method according to any of the preceding claims, wherein the subseries is automatically terminated if the user has failed incorrectly identifying the orientation for at least one grating pattern (4,5)

   and/or wherein the electronic device is a portable electronic device (1), and wherein preferably the display (2) is a touch sensitive display
   and/or wherein the grating patterns (4, 5) are sinusoidal grating patterns (4, 5).

10. Method according to any of the preceding claims, wherein the electronic device (1) comprises at least one camera (8) facing in the direction of the display and of the user, and wherein said camera (8) is used for controlling the method, wherein said control comprises at least one of the following:

    control of the distance of the eye of the user to the display, control of the open eye of the user, control of the identity of the user, preferably by using face recognition,
    wherein preferably the output of the camera is used for controlling at least one of the following parameters of the method: subseries, type and number per subseries of grating pattern (4, 5) (colour, monochromatic), spatial frequency of the grating patterns (4, 5), temporal speed of switching between grating patterns (4, 5) along the subseries, degree of change of contrast between grating patterns (4, 5) between grating patterns.

11. Method according to any of the preceding claims, wherein the contrast amplitude of the sinusoidal grating pattern (4,5) within each subseries is successively decreasing, and wherein this decrease is linear along each full subseries or is linear in an initial phase until reaching a switch point, preferably statically determined or as a function of the behaviour of the user, and subsequently changed to a different less steep linear or non-linear decrease.

12. Method according to any of the preceding claims, wherein the spatial frequency of the preferably sinusoidal grating patterns (4, 5) is in the range of 0.01-5 lines/mm, preferably in the range of 0.3-3 lines/mm, and wherein preferably within one subseries or within all subseries the spatial frequency is constant.

13. Method according to any of the preceding claims, wherein on the display where the grating patterns (4, 5) are displayed, there are displayed input fields or buttons (9-11) for the user to touch manually for signing the respective grating pattern (4, 5) a perceived orientation, wherein preferably there is at least one button (9) for indicating a vertical orientation, preferably located above or below the grating pattern, a separate button (10) for indicating a left orientation, preferably located to the left of the grating pattern, and a separate button (11) for indicating a right orientation, preferably located to the right of the grating pattern, or a single slide button for inputting the perceived orientation is provided.

14. Method according to any of the preceding claims, wherein the grating pattern (4, 5) is rectangular, square, oval or circular field, preferably a circular field.

15. Data processing program for carrying out a method according to any one of claims 1-14 on a preferably portable electronic device (1) having a display (2) and a data evaluation unit, wherein the display (3) is preferably a touch-sensitive display, and wherein furthermore preferably the electronic device additionally has a camera directed towards the user, wherein the display grating patterns (4, 5) is two- or three-dimensional, preferably in the form of a mobile application.

FIG. 1

Europäisches
Patentamt

European
Patent Office

Office européen
des brevets

# EUROPEAN SEARCH REPORT

Application Number

EP 22 21 4490

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/174284 A1 (STEWART JEFFREY L [US]) 18 September 2003 (2003-09-18) | 1-9, 11-15 | INV. A61B3/02 |
| Y | * paragraphs [0018] – [0027] * | 10 | |
| X | US 2019/387967 A1 (VALENTI DENISE A [US]) 26 December 2019 (2019-12-26) * paragraphs [0032], [0035], [0067] – [0091] * | 1-9, 11-15 | |
| Y | WO 2021/130743 A1 (SHAMIR OPTICAL IND LTD [IL]) 1 July 2021 (2021-07-01) * paragraphs [0021], [0045] * | 10 | |

TECHNICAL FIELDS
SEARCHED      (IPC)

A61B

The present search report has been drawn up for all claims

| Place of search | Date of completion of the search | Examiner |
|---|---|---|
| Munich | 8 May 2023 | Martelli, Luca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 388 977 A1**

## ANNEX TO THE EUROPEAN SEARCH REPORT
## ON EUROPEAN PATENT APPLICATION NO.

EP 22 21 4490

08-05-2023

| Patent document cited in search report | | Publication date | Patent family member(s) | | Publication date |
|---|---|---|---|---|---|
| US 2003174284 | A1 | 18-09-2003 | NONE | | |
| US 2019387967 | A1 | 26-12-2019 | US 2017105669 | A1 | 20-04-2017 |
| | | | US 2019387967 | A1 | 26-12-2019 |
| | | | US 2020337550 | A1 | 29-10-2020 |
| | | | US 2022104702 | A1 | 07-04-2022 |
| WO 2021130743 | A1 | 01-07-2021 | AU 2020412896 | A1 | 09-06-2022 |
| | | | CA 3159435 | A1 | 01-07-2021 |
| | | | CN 114828731 | A | 29-07-2022 |
| | | | EP 4081094 | A1 | 02-11-2022 |
| | | | US 10827918 | B1 | 10-11-2020 |
| | | | WO 2021130743 | A1 | 01-07-2021 |